# FASCICULE DE BREVET EUROPEEN

(11) **EP 3 285 654 B1**
(45) Date de publication et mention de la délivrance du brevet: **25.08.2021**
(21) Numéro de dépôt: 16722301.5
(22) Date de dépôt: 22.04.2016
(51) Int. Cl.: A61B 10/00

(54) **PROCÉDÉ DE PRÉLÈVEMENT DE MICRO-ORGANISMES, DISPOSITIF DE PRÉLÈVEMENT DE MICRO-ORGANISMES ET KIT DE PRÉLÈVEMENT COMPRENANT UN TEL DISPOSITIF DE PRÉLÈVEMENT**
VERFAHREN ZUR PROBENENTNAHME VON MIKROORGANISMEN, VORRICHTUNG ZUR PROBENENTNAHME VON MIKROORGANISMEN UND KIT MIT SOLCHER VORRICHTUNG
PROCEDURE FOR THE SAMPLING OF MICROORGANISMS, DEVICE FOR THE SAMPLING OF MICROORGANISMS AND KIT COMPRISING SAID DEVICE

(30) Priorité: 24.04.2015 FR 1553721
(43) Date de publication de la demande: 28.02.2018
(73) Titulaire: Maat Pharma, 69007 Lyon (FR); Institut national de recherche pour l'agriculture, l'alimentation et l'environnement, 75007 Paris (FR)
(72) Inventeur: SCHWINTNER, Carole, 69007 Lyon (FR); AFFAGARD, Hervé, 69007 Lyon (FR); DORE, Joël, 94400 Vitry-sur-Seine (FR)
(74) Mandataire: Santarelli
(86) Numéro de dépôt international: PCT/FR2016/050963
(87) Numéro de publication internationale: WO 2016/170290

(56) Documents cités:
- WO-A1-2011/122949
- WO-A1-2013/090825
- US-A1- 2011 020 860

## Description

L'invention se rapporte à un procédé de prélèvement de micro-organismes, à un dispositif de prélèvement de micro-organismes et à un kit de prélèvement mettant en œuvre un tel dispositif de prélèvement.

L'invention s'applique notamment au prélèvement de microbiote intestinal humain par exemple pour traiter les dysbioses intestinales, telles que les infections à *Clostridium difficile,* par transplantation du microbiote intestinal.

Le microbiote intestinal est constitué par l'ensemble des micro-organismes (bactéries, levures et champignons) qui se trouvent dans le système gastro-intestinal humain (intestin, estomac et selles). La diversité microbienne est estimée à l'heure actuelle à environ 10³ espèces bactériennes composant le microbiote intestinal dominant d'un individu adulte, avec une abondance de 10¹⁴ bactéries, représentant un métagénome bactérien de 200 000 à 800 000 gènes chez chaque individu, soit 10 à 50 fois le nombre de gènes du génome humain.

Stérile *in utero,* l'intestin se colonise dès les premiers jours de vie jusqu'à évoluer vers un microbiote individuel unique. Chaque personne possède des bactéries relativement proches en termes d'espèces mais la composition exacte de son microbiote (espèces, proportions) est pour une large part (environ ²/₃ des espèces) spécifique de l'hôte.

Ainsi, le microbiote intestinal humain est un écosystème très diversifié, complexe et spécifique de chaque individu.

Il est essentiel pour la santé d'un individu de maintenir un microbiote stable qui est à la fois capable de revenir à son état initial après un changement et résistant à l'invasion. Le maintien d'une grande diversité du microbiote favorise sa stabilité.

Cependant, certaines pathologies ou traitements déséquilibrent le microbiote : par exemple, les antibiotiques ainsi que les maladies à composante inflammatoire, telles que la maladie inflammatoire chronique de l'intestin (MICI), peuvent limiter la diversité du microbiote dans l'intestin. Les traitements antibiotiques (ou antibiothérapie), notamment, résultent en une altération du microbiote, ce qui peut favoriser la prolifération d'organismes pathogènes comme *Clostridium difficile.* Les infections à *Clostridium difficile* sont responsables de la diarrhée nosocomiale ; cette bactérie est résistante à l'antibiothérapie classique (à spectre large, tels que vancomycine et métronidazole).

Afin de rétablir la flore intestinale et lutter contre les infections de type *Clostridium difficile,* et ainsi rétablir l'homéostasie *(i.e.* la symbiose), une transplantation de microbiote fécal est envisagée et testée. Elle consiste en l'introduction des selles d'un sujet donneur sain dans le tube digestif d'un patient receveur, afin de rééquilibrer la flore intestinale altérée de l'hôte. Cette transplantation de microbiote fécal peut être allogénique (c'est-à-dire d'un individu donneur sain vers un patient) ou autologue (c'est-à-dire d'un individu vers lui-même). Les résultats obtenus sur les infections de type *Clostridium difficile* sont encourageants, et certains patients ont été traités avec succès (Tauxe et al, Lab Medicine, Winter 2015, volume 46, Number 1).

Préalablement à la réalisation d'une telle transplantation, le microbiote est généralement prélevé chez le sujet donneur sain par l'intermédiaire d'un dispositif de prélèvement du type comprenant :
- un conteneur comprenant un corps qui comporte un espace intérieur adapté pour recevoir la matière biologique, et un col qui délimite une ouverture d'accès à l'espace intérieur du corps, le corps du conteneur étant constitué d'une poche souple,
- un couvercle adapté pour être monté de manière amovible et étanche sur le col du conteneur de manière à obturer l'ouverture d'accès du col et à fermer l'espace intérieur du corps,
- un organe de transfert comportant intérieurement un passage entre l'espace intérieur et un environnement extérieur, l'organe de transfert présentant un état ouvert dans lequel ledit organe de transfert établit une communication avec l'espace intérieur au travers du passage, et un état fermé dans lequel ledit organe de transfert empêche toute communication avec l'espace intérieur au travers du passage.

Un dispositif de prélèvement connu de ce type est décrit dans le document WO 2013/090825. Avec un tel dispositif de prélèvement, après avoir collecté la matière biologique, le conteneur est fermé par le couvercle avant de mettre la matière biologique en suspension en mélangeant la matière biologique collectée avec un fluide diluant. Des conditions anaérobies peuvent être obtenues dans l'espace intérieur du corps par une aspiration, éventuellement complétée par l'ajout de substances appropriées.

Toutefois, le dispositif de prélèvement connu n'offre pas les conditions permettant d'assurer de manière simple une préservation satisfaisante des bactéries du microbiote et, tout particulièrement des bactéries anaérobies, composants majoritaires du microbiote intestinal. En outre, le dispositif de prélèvement connu ne permet pas de réaliser les manipulations nécessaires au prélèvement du microbiote de manière sécurisée, l'opérateur réalisant les manipulations risquant d'entrer en contact avec la matière biologique.

L'invention vise à pallier les problèmes évoqués ci-dessus.

A cet effet, selon un premier aspect, l'invention propose un procédé de prélèvement de micro-organismes contenus dans une matière biologique, le procédé de prélèvement mettant en œuvre un dispositif de prélèvement du type précité dans lequel ledit au moins un organe de transfert comprend un organe d'évacuation adapté pour, dans l'état ouvert, évacuer au moins une partie des gaz contenus dans l'espace intérieur du corps du conteneur, le procédé de prélèvement comprenant les étapes consistant à :
- collecter la matière biologique dans l'espace intérieur du corps du conteneur, et fermer l'espace intérieur en montant le couvercle sur le col du conteneur,
- mettre l'espace intérieur du corps du conteneur en anaérobiose en mettant l'organe d'évacuation dans l'état ouvert, en comprimant le corps du conteneur de manière à évacuer au moins une partie des gaz contenus dans l'espace intérieur du corps du conteneur et en mettant l'organe d'évacuation dans l'état fermé.

Ainsi, l'invention permet d'obtenir de manière simple et sécurisée des conditions optimales de préservation des micro-organismes et en particulier du microbiote. En particulier, l'invention permet de réaliser une mise en anaérobiose par une simple compression exercée sur le corps du conteneur pour évacuer l'oxygène et conserver les bactéries anaérobies sensibles à l'oxygène. Les bactéries maintenues dans un système clos peuvent être conservées dans ces conditions d'anaérobiose en étant isolées des contaminants extérieurs. De plus, cette mise en anaérobiose ainsi que la mise en suspension ultérieure de la matière biologique réalisées en comprimant le corps du conteneur, sans avoir recours à un mixeur, permettent d'augmenter la viabilité des bactéries. En outre, les manipulations peuvent être réalisées avec le conteneur fermé par le couvercle et donc sans risque de contact direct avec la matière biologique pour l'opérateur.

L'organe d'évacuation peut présenter des faces intérieure et extérieure opposées, l'organe d'évacuation étant dans l'état fermé au repos et passant à l'état ouvert lorsqu'une différence de pression est appliquée entre les faces intérieure et extérieure. Le procédé de prélèvement peut alors prévoir, au cours de l'étape consistant à mettre l'espace intérieur du corps du conteneur en anaérobiose, de mettre automatiquement l'organe d'évacuation dans l'état ouvert en comprimant le corps du conteneur puis de mettre automatiquement l'organe d'évacuation dans l'état fermé en arrêtant de comprimer le corps du conteneur.

Le couvercle peut comporter une partie d'appui présentant une rigidité d'ensemble et l'organe d'évacuation peut être ménagé dans la partie d'appui du couvercle. Le procédé de prélèvement peut alors prévoir, au cours de l'étape consistant à mettre l'espace intérieur du corps du conteneur en anaérobiose, de presser le corps du conteneur contre la partie d'appui du couvercle.

Le procédé de prélèvement peut comprendre en outre l'étape consistant à mettre en suspension la matière biologique en introduisant un fluide diluant dans l'espace intérieur du corps du conteneur par l'intermédiaire dudit au moins un organe de transfert dans l'état ouvert, et en mélangeant la matière biologique et le fluide diluant par pression sur le corps du conteneur.

Le procédé de prélèvement peut comprendre en outre l'étape consistant à prélever les micro-organismes en recueillant au moins une partie de la matière biologique mise en suspension par l'intermédiaire dudit au moins un organe de transfert dans l'état ouvert.

Le procédé de prélèvement peut prévoir, au cours de l'étape consistant à collecter la matière biologique dans l'espace intérieur du corps du conteneur, de collecter directement de la matière fécale en plaçant le dispositif de prélèvement sur un siège de toilettes.

Selon un deuxième aspect, l'invention propose un dispositif de prélèvement de micro-organismes contenus dans une matière biologique, le dispositif de prélèvement comprenant :
- un conteneur comprenant un corps qui comporte un espace intérieur adapté pour recevoir la matière biologique, et un col qui délimite une ouverture d'accès à l'espace intérieur du corps, le corps du conteneur étant constitué d'une poche souple,
- un couvercle adapté pour être monté de manière amovible et étanche sur le col du conteneur de manière à obturer l'ouverture d'accès du col et à fermer l'espace intérieur du corps,
dans lequel au moins l'un parmi le conteneur et le couvercle est pourvu d'au moins un organe de transfert comportant intérieurement un passage entre l'espace intérieur et un environnement extérieur, l'organe de transfert présentant un état ouvert dans lequel ledit organe de transfert établit une communication avec l'espace intérieur au travers du passage, et un état fermé dans lequel ledit organe de transfert empêche toute communication avec l'espace intérieur au travers du passage,
dans lequel ledit au moins un organe de transfert comprend un organe d'évacuation présentant des faces intérieure et extérieure opposées, l'organe d'évacuation étant dans l'état fermé au repos et passant à l'état ouvert lorsqu'une différence de pression est appliquée entre les faces intérieure et extérieure, de manière à mettre automatiquement l'organe d'évacuation dans l'état ouvert en comprimant le corps du conteneur pour évacuer au moins une partie des gaz contenus dans l'espace intérieur du corps du conteneur et mettre l'espace intérieur du corps du conteneur en anaérobiose, et à mettre automatiquement l'organe d'évacuation dans l'état fermé en arrêtant de comprimer le corps du conteneur.

Pour éviter la dissémination des germes et donc garantir la sécurité du manipulateur, l'organe d'évacuation peut comprendre une membrane de retenue microporeuse disposée dans le passage et adaptée pour retenir la matière biologique tout en laissant passer les gaz.

Ledit au moins un organe de transfert peut comprendre un port de connexion ménagé au travers de l'un parmi le conteneur et le couvercle et comportant intérieurement au moins une partie du passage, et un élément d'obturation déplaçable par rapport au port de connexion entre une position d'obturation, dans laquelle ledit élément d'obturation empêche toute communication au travers du passage, et une position de libération dans laquelle ledit élément d'obturation autorise la communication au travers du passage.

L'élément d'obturation peut être monté dans le passage du port de connexion, l'élément d'obturation étant sollicité vers la position d'obturation et étant déplaçable vers la position de libération. Ces dispositions permettent de réaliser de manière simple une connexion sans perçage, amovible et étanche autorisant, le cas échéant, une communication bidirectionnelle tout en maintenant l'espace intérieur parfaitement clos tant que l'élément d'obturation n'est pas déplacé par une action positive vers la position de libération.

Ledit au moins un organe de transfert peut comprendre au moins un tube s'étendant depuis le port de connexion et comportant intérieurement une partie du passage.

Ledit au moins un organe de transfert peut comprendre au moins une vanne montée sur le tube, la vanne étant déplaçable entre une position fermée, dans laquelle ladite vanne empêche toute communication au travers du passage, et une position ouverte dans laquelle ladite vanne autorise la communication au travers du passage.

Lorsque l'élément d'obturation est monté dans le passage du port de connexion, le tube peut comporter un port de connexion complémentaire pourvu d'un élément de libération et adapté pour être connecté de manière amovible au port de connexion de telle manière que l'élément de libération déplace l'élément d'obturation vers la position de libération.

Le couvercle peut comporter une partie d'appui présentant une rigidité d'ensemble et l'organe d'évacuation peut être ménagé dans la partie d'appui du couvercle, de manière à pouvoir presser le corps du conteneur contre la partie d'appui du couvercle pour mettre l'espace intérieur du corps du conteneur en anaérobiose.

Le dispositif de prélèvement peut comprendre en outre un filtre solidarisé au conteneur de manière à définir, dans l'espace intérieur, un compartiment supérieur dans lequel débouche l'ouverture d'accès, et un compartiment inférieur, le filtre présentant des pores compris entre 0,1 mm et 1,5 mm.

Afin d'éviter une occlusion du passage par un corps solide contenu dans la matière biologique ou un prélèvement d'un tel corps solide avec les micro-organismes, ledit au moins un organe de transfert peut être prévu dans le compartiment inférieur, en aval du filtre par rapport à l'ouverture d'accès.

Selon un troisième aspect, l'invention concerne un kit de prélèvement comprenant :
- un dispositif de prélèvement tel que défini précédemment, et
- au moins un dispositif annexe choisi parmi un dispositif d'alimentation adapté pour alimenter l'espace intérieur en fluide, et un dispositif de réception adapté pour recevoir un fluide contenu dans l'espace intérieur, le dispositif annexe étant notamment choisi parmi un réservoir de fluide diluant, un tube d'analyse, une tubulure de distribution et une poche de recueil de micro-organismes.

Le kit de prélèvement permet notamment de prélever et de conditionner les micro-organismes sous forme d'inoculum prêt à l'emploi.

Lorsque l'organe de transfert du dispositif de prélèvement comprend un élément d'obturation monté dans le passage d'un port de connexion, le dispositif annexe peut comporter un port de connexion complémentaire pourvu d'un élément de libération et adapté pour être connecté de manière amovible au port de connexion de telle manière que l'élément de libération déplace l'élément d'obturation vers la position de libération.

D'autres objets et avantages de l'invention apparaîtront à la lecture de la description qui suit d'un mode de réalisation particulier de l'invention donné à titre d'exemple non limitatif, la description étant faite en référence aux dessins annexés dans lesquels :

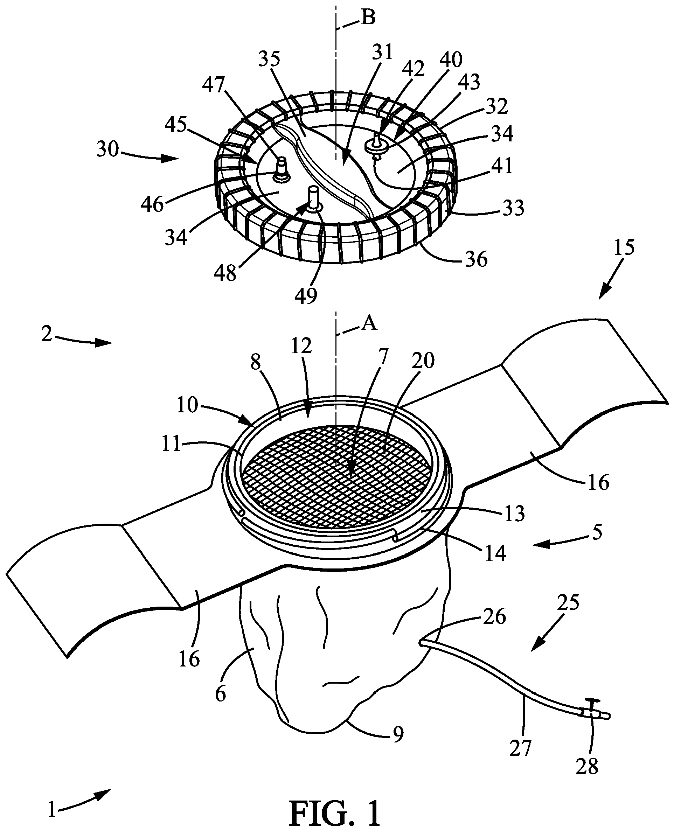

- la figure 1 est une représentation en perspective d'un kit de prélèvement comprenant un dispositif de prélèvement de microbiote contenu dans une matière biologique, le dispositif de prélèvement comprenant un conteneur et un couvercle, le conteneur comprenant un corps constitué d'une poche souple et le couvercle étant pourvu d'un organe d'évacuation adapté pour évacuer au moins une partie des gaz contenus dans un espace intérieur du corps du conteneur,

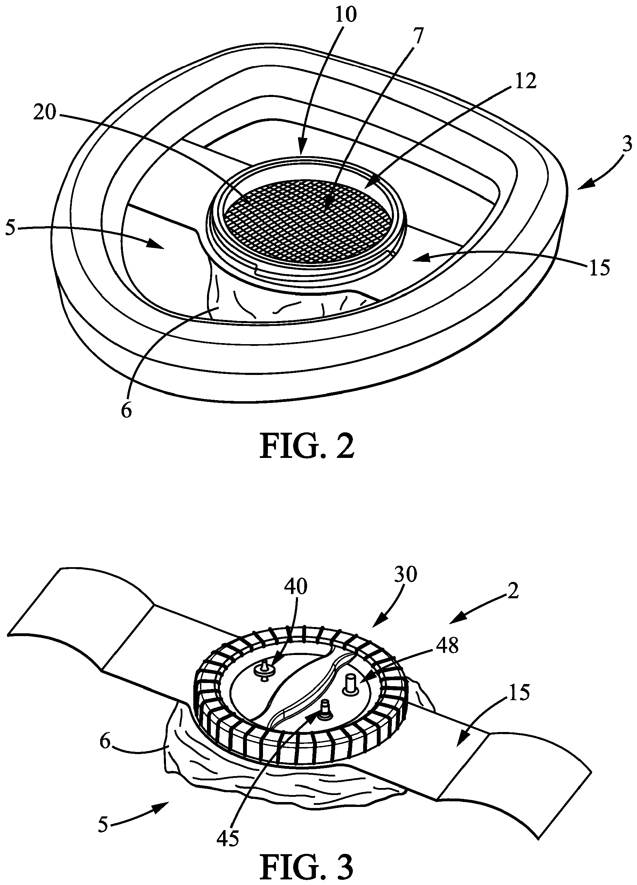

- la figure 2 est une représentation en perspective du dispositif de prélèvement de la figure 1 dans une application à un prélèvement de microbiote intestinal contenu dans de la matière fécale comme matière biologique, le dispositif de prélèvement étant ouvert avec le couvercle séparé du conteneur pour collecter de la matière fécale,
- la figure 3 est une représentation en perspective du dispositif de prélèvement de la figure 1 mis en condition d'anaérobiose avec le couvercle monté sur le conteneur et le corps pressé contre le couvercle pour évacuer l'oxygène de l'espace intérieur du corps et conserver le microbiote présent dans la matière fécale collectée,

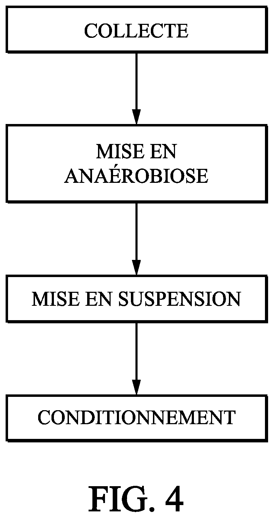

- la figure 4 est un organigramme illustrant des étapes d'un procédé de prélèvement de microbiote contenu dans une matière biologique, le procédé de prélèvement mettant en œuvre le dispositif de prélèvement de la figure 1.

Sur les figures, les mêmes références désignent des éléments identiques ou analogues.

La figure 1 représente un mode de réalisation d'un kit de prélèvement 1 comprenant un dispositif de prélèvement 2 de micro-organismes contenus dans une matière biologique. Dans le mode de réalisation représenté, sans y être limité, le dispositif de prélèvement 2 est mis en œuvre dans le prélèvement de microbiote intestinal contenu dans de la matière fécale d'un individu.

Le dispositif de prélèvement 2 comprend un conteneur 5 destiné notamment à collecter de la matière fécale.

Le conteneur 5 comprend un corps 6 constitué d'une poche souple délimitant un espace intérieur 7 adapté pour recevoir la matière biologique et présentant un bord supérieur 8. Le corps 6 est réalisé en un matériau déformable adapté pour former une barrière à la matière fécale contenue dans l'espace intérieur 7 et aux fluides extérieurs afin d'éviter tout échange entre la matière fécale collectée et l'extérieur. En particulier, le corps 6 peut être réalisé en polyéthylène (PE), en polypropylène (PP), en polychlorure de vinyle (PVC), en polycarbonate (PC) ou en éthylène acétate de vinyle (EVA).

Le conteneur 5 comprend également un col 10 cylindrique de révolution autour d'un axe central A auquel le bord supérieur 8 du corps 6 est solidarisé par tout moyen approprié et notamment par soudage, collage ou autre. Le col 10 présente une rigidité d'ensemble et est, par exemple, réalisé en matière plastique telle que du polyéthylène (PE), du polypropylène (PP), du polychlorure de vinyle (PVC), du polycarbonate (PC) ou du éthylène acétate de vinyle (EVA). Le col 10 présente une surface intérieure 11 qui délimite une ouverture d'accès 12 à l'espace intérieur 7 du corps 6, et une surface extérieure 13 pourvue d'une ou plusieurs rampes 14 agencées de manière à former un filet externe. En variante, tout autre agencement continu ou discret d'une ou plusieurs rampes 14, éventuellement équiréparties, pourrait être prévu sur la surface extérieure 13 du col 10.

Le conteneur 5 comporte également un support 15 adapté pour en assurer un maintien stable dans une orientation appropriée et, en particulier, avec l'ouverture d'accès 12 orientée vers le haut. Dans le mode de réalisation représenté, le support 15 est solidaire du col 10 et conformé pour pouvoir venir en appui sur un rebord d'un siège de toilettes 3 conformément à l'application considérée. Le support 15 comprend deux ailes 16 s'étendant radialement par rapport à l'axe central A dans des sens diamétralement opposés depuis la surface extérieure 13 du col 10. En variante, selon l'application, le support pourrait avoir toute autre forme appropriée.

Un filtre 20 est solidarisé, par exemple par soudage, collage ou autre, au conteneur 5 de manière à définir, dans l'espace intérieur 7, deux compartiments. Sur la figure 1, le filtre 20 est solidarisé au corps 6 de manière à s'étendre au voisinage de l'ouverture d'accès 12 du col 10. L'espace intérieur 7 présente alors un compartiment supérieur dans lequel débouche l'ouverture d'accès 12 et un compartiment inférieur du côté du fond 9. En variante, le filtre peut être solidarisé en tout autre emplacement approprié du col ou du corps du conteneur pour définir des compartiments inférieur et supérieur de contenances respectives appropriées. Le filtre présente des pores compris entre 0,1 mm et 1,5 mm, notamment entre 0,3 mm et 0,5 mm, adaptés pour retenir des corps solides non solubles contenus dans la matière fécale.

Le corps 6 du conteneur 5 comprend un premier organe de transfert formant, dans le mode de réalisation particulier représenté, un organe de recueil 25 permettant de recueillir les micro-organismes du microbiote intestinal. L'organe de recueil 25 comporte un port de connexion inférieur 26 ménagé au travers du corps 6 du conteneur 5 au voisinage du fond 9 du corps 6, et un tube 27 s'étendant depuis le port de connexion inférieur 26. Le port de connexion inférieur 26 et le tube 27 définissent ainsi un passage à l'intérieur de l'organe de recueil 25 entre l'espace intérieur 7 et un environnement extérieur. L'organe de recueil 25 comporte une vanne 28 montée sur le tube 27 et déplaçable entre :
- une position fermée définissant un état fermé de l'organe de recueil 25 et dans laquelle elle empêche toute communication, et notamment toute circulation de fluide, au travers du passage, et
- une position ouverte définissant un état ouvert de l'organe de recueil 25 et dans laquelle elle autorise la communication, et notamment la circulation de fluide, au travers du passage.

En variante, l'organe de recueil 25 pourrait comprendre plusieurs tubes 27 et plusieurs vannes 28.

Le tube 27 peut être relié de manière amovible au port de connexion inférieur 26. Un élément d'obturation sous la forme d'un clapet peut être monté dans le passage du port de connexion inférieur 26 en étant sollicité vers une position d'obturation, dans laquelle l'élément d'obturation empêche toute communication au travers du passage, et en étant déplaçable vers une position de libération dans laquelle l'élément d'obturation autorise la communication au travers du passage. Le tube 27 comporte alors un port de connexion complémentaire adapté pour coopérer avec le port de connexion inférieur 26. En particulier, le port de connexion complémentaire est pourvu d'un élément de libération agencé pour déplacer l'élément d'obturation vers la position de libération lorsque le port de connexion complémentaire est connecté au port de connexion inférieur 26. Le port de connexion inférieur 26 et le port de connexion complémentaire peuvent alors former, l'un, l'organe mâle et, l'autre, l'organe femelle d'une connexion de type Luer Lock équipée d'un clapet et dans laquelle :
- le port de connexion inférieur 26 est dans la position d'obturation empêchant toute communication entre l'espace intérieur 7 et l'extérieur au travers du passage lorsque le tube 27 est séparé du corps 6 du conteneur 5, et
- le port de connexion inférieur 26 est dans la position de libération établissant une communication entre l'espace intérieur 7 et l'extérieur au travers du passage lorsque le tube 27 est relié au corps 6 du conteneur 5.

Un tel port de connexion permet, par une connexion sans perçage, de réaliser une communication bidirectionnelle au travers du passage tout en maintenant l'espace intérieur parfaitement clos tant que l'élément d'obturation n'est pas déplacé vers la position de libération par l'élément de libération.

Dans un autre mode de réalisation, le tube 27 de l'organe de recueil 25 peut être venu de matière avec le port de connexion inférieur 26, la vanne 28 formant alors un élément d'obturation déplaçable par rapport au port de connexion 26 entre une position d'obturation correspondant à la position fermée de la vanne 28, et une position de libération correspondant à la position ouverte. En variante, en remplacement de la vanne 28, l'élément d'obturation pourrait être un bouchon transperçable ou monté de manière amovible sur une extrémité libre du tube 27.

Afin de pouvoir conserver la matière fécale collectée, le dispositif de prélèvement 2 comprend un couvercle 30 adapté pour être monté de manière amovible et étanche sur le col 10 du conteneur 5 de manière à obturer l'ouverture d'accès 12 du col 10 et à fermer l'espace intérieur 7 du corps 6. Le couvercle 30 présente une rigidité d'ensemble et est, par exemple, réalisé en une matière plastique analogue à celle du col 10. Il comporte une paroi transversale 31 s'étendant perpendiculairement à un axe central B et présentant un bord périphérique 32 circulaire avec un diamètre correspondant à celui du col 10 du conteneur 5. Le couvercle 30 comporte également une jupe 33 cylindrique de révolution autour de l'axe central B qui s'étend depuis le bord périphérique 32 de la paroi transversale 31. La jupe 33 présente une surface intérieure pourvue de saillies formant, le cas échéant, un filet interne, adaptées pour coopérer avec les rampes 14 sur la surface extérieure 13 du col 10. En variante, tout autre dispositif permettant de solidariser de manière amovible et étanche le couvercle 30 et le col 10 du conteneur 5 pourrait être prévu et, notamment, un dispositif de clipsage. La paroi transversale 31 a une surface supérieure présentant deux évidements 34 agencés de part et d'autre d'une saillie de saisie 35 centrale. Les évidements 34 sont tels qu'une surface inférieure de la paroi transversale 31 au niveau des évidements 34 affleure ou dépasse d'un bord libre 36 de la jupe 33.

Dans l'un des évidements 34, le couvercle 30 est pourvu d'un deuxième organe de transfert réalisé sous la forme d'un organe d'évacuation adapté pour, dans un état ouvert, évacuer au moins une partie des gaz contenus dans l'espace intérieur 7 du corps 6 du conteneur 5. Dans le mode de réalisation particulier représenté, l'organe d'évacuation est une valve anti-retour 40 qui est dans un état fermé au repos, en l'absence de sollicitation extérieure, et qui passe à un état ouvert lorsqu'une différence de pression est appliquée entre des faces intérieure et extérieure opposées, situées respectivement du côté des surfaces intérieure et extérieure du couvercle 30.

En particulier, la valve anti-retour 40 comprend un port de connexion supérieur 41 ménagé au travers du couvercle 30, entre ses surfaces inférieure et supérieure, et un tube 42 s'étendant depuis le port de connexion supérieur 41. Le port de connexion supérieur 41 et le tube 42 définissent ainsi un passage à l'intérieur de la valve anti-retour 40 entre l'espace intérieur 7 et l'environnement extérieur. Dans un bourrelet central 43, le tube 42 de la valve anti-retour 40 comprend une membrane de retenue microporeuse disposée dans le passage et adaptée pour retenir la matière biologique tout en laissant passer les gaz. La membrane de retenue microporeuse peut notamment être du type de celle commercialisée par la société POREX ® FILTRATION sous la référence XS-49 110 en polypropylène avec des pores compris entre 125 µm et 175 µm. En variante, la membrane de retenue microporeuse pourrait être réalisée de toute autre manière appropriée et notamment en un matériau fritté présentant des pores compris entre 100 µm et 350 µm, notamment entre 200 µm et 300 µm, par exemple de 250 µm.

Un élément d'obturation sous la forme d'un clapet peut être monté dans le passage de la valve anti-retour 40 en étant sollicité vers une position d'obturation, empêchant toute communication au travers du passage, et en étant déplaçable vers une position de libération autorisant une communication au travers du passage.

Dans le mode de réalisation représenté, le tube 42 de la valve anti-retour 40 est venu de matière avec le port de connexion supérieur 41. L'élément d'obturation peut être prévu dans le tube 42, notamment au voisinage d'une extrémité libre.

Dans un autre mode de réalisation, le tube 42 de la valve anti-retour 40 peut être relié de manière amovible au port de connexion supérieur 41. En complément ou en remplacement du clapet prévu dans le tube 42, un nouvel élément d'obturation sous la forme d'un clapet peut être prévu dans le port de connexion supérieur 41 en étant sollicité vers une position d'obturation, empêchant toute communication au travers du passage, et en étant déplaçable vers une position de libération autorisant une communication au travers du passage. Dans cet autre mode de réalisation, le tube 42 comporte un port de connexion complémentaire adapté pour coopérer avec le port de connexion supérieur 41. En particulier, le port de connexion complémentaire est pourvu d'un élément de libération agencé pour déplacer le clapet du port de connexion supérieur 41 vers la position de libération lorsque le port de connexion complémentaire est connecté au port de connexion supérieur 41. Le port de connexion supérieur 41 est dans la position d'obturation lorsque le tube 42 est séparé du couvercle 30, et dans la position de libération lorsque le tube 42 est relié au couvercle 30.

En variante, l'organe d'évacuation pourrait être réalisé de toute autre manière appropriée qu'une valve anti-retour. En particulier, l'organe d'évacuation pourrait comporter un passage pouvant être obturé par un élément d'obturation sous la forme d'un bouchon placé de manière amovible dans le passage, par exemple en étant monté de manière amovible sur l'extrémité libre du tube 42. Lorsque le bouchon est écarté du passage, l'organe d'évacuation est dans l'état ouvert, dans lequel une communication entre l'espace intérieur 7 du conteneur 5 et l'extérieur est établie au travers du passage. Lorsque le bouchon est placé dans le passage, il obture le passage et l'organe d'évacuation est dans un état fermé, dans lequel toute communication entre l'espace intérieur 7 du conteneur 5 et l'extérieur au travers du passage est empêchée.

Dans l'autre évidement 34, le couvercle 30 comprend des troisième et quatrième organes de transfert formant, dans le mode de réalisation particulier représenté, respectivement un organe d'alimentation 45 et un organe de prélèvement 48. Les organes d'alimentation 45 et de prélèvement 48 sont adaptés chacun pour établir une communication entre l'espace intérieur et l'extérieur dans l'état ouvert, et pour fermer la communication entre l'espace intérieur et l'extérieur dans l'état fermé.

Les organes de d'alimentation 45 et de prélèvement 48 comprennent respectivement des ports de connexion supérieurs supplémentaires 46, 49 pour des dispositifs annexes du kit de prélèvement.

Chacun des ports de connexion supérieurs supplémentaires 46, 49 est ménagé au travers du couvercle 30, entre ses surfaces inférieure et supérieure, pour constituer l'un des organes, par exemple femelle, d'une connexion de type Luer Lock. Comme décrit précédemment, chacun des ports de connexion supérieurs supplémentaires 46, 49 des organes d'alimentation 45 et de prélèvement 48 peut être pourvu d'un élément d'obturation réalisé sous la forme d'un clapet 47, d'un bouchon ou autre.

L'organe d'obturation de l'organe d'alimentation 45 est, par exemple, réalisé sous la forme d'un clapet 47 monté dans le passage et adapté pour coopérer avec un élément de libération d'un port de connexion complémentaire prévu sur le dispositif annexe et constituant l'autre organe, par exemple mâle, de la connexion de type Luer Lock. Le clapet 47 sollicité vers une position d'obturation empêchant toute communication au travers du passage place le port de connexion supérieur supplémentaire 46 de l'organe d'alimentation 45 dans un état fermé lorsque le dispositif annexe est séparé du couvercle 30. L'élément d'obturation 47 déplacé vers une position de libération autorisant une communication au travers du passage place le port de connexion supérieur supplémentaire 46 de l'organe d'alimentation 45 dans un état ouvert lorsque le dispositif annexe est relié au couvercle 30.

L'organe d'obturation de l'organe de prélèvement 48 est, par exemple, réalisé sous la forme d'un bouchon, non représenté, monté de manière amovible sur le port de connexion supérieur supplémentaire 49. L'organe de prélèvement 48 est dans un état ouvert autorisant une communication au travers du passage lorsque le bouchon est séparé du port de connexion supérieur supplémentaire 49, et dans un état fermé empêchant toute communication au travers du passage lorsque le bouchon est sur le port de connexion supérieur supplémentaire 49.

Les dispositifs annexes peuvent notamment comprendre :
- un ou plusieurs réservoirs de fluide diluant, et notamment une poche d'alimentation, adaptés pour être reliés au port de connexion supérieur supplémentaire 46 de l'organe d'alimentation 45, le réservoir comprenant, le cas échéant, une conduite équipé d'un port de connexion complémentaire avec un élément de libération,
- un ou plusieurs tubes d'analyse adaptés pour être reliés au port de connexion supérieur supplémentaire 49 de l'organe de prélèvement 48, chaque tube d'analyse s'étendant entre une extrémité ouverte pourvue, le cas échéant, d'un port de connexion complémentaire avec un élément de libération, et une extrémité fermée de manière à pouvoir extraire à des fins d'analyse de la matière fécale, et
- une ou plusieurs poches de recueil de micro-organismes adaptées pour être reliées au port de connexion inférieur 26 de l'organe de recueil 25 par l'intermédiaire d'une tubulure de distribution comprenant une ou plusieurs conduites équipée d'un port de connexion complémentaire avec un élément de libération et, le cas échéant, d'une ou plusieurs vannes.

En variante, le kit de prélèvement 1 pourrait comprendre tout autre type de dispositif annexe choisi parmi un dispositif d'alimentation adapté pour alimenter l'espace intérieur 7 en fluide, et un dispositif de réception adapté pour recevoir un fluide contenu dans l'espace intérieur 7.

L'invention a été décrite avec un dispositif de prélèvement comprenant des premier, deuxième, troisième et quatrième organes de transfert constituant respectivement un organe de recueil, un organe d'évacuation sous la forme d'une valve anti-retour, un organe d'alimentation et un organe de prélèvement. En variante, il pourrait être prévu tout autre agencement, toute autre réalisation et toute autre fonctionnalité d'un ou plusieurs organes de transfert comportant intérieurement un passage et dont au moins l'un forme un organe d'évacuation adapté pour, dans l'état ouvert, évacuer au moins une partie des gaz contenus dans l'espace intérieur du corps du conteneur. En particulier, le dispositif de prélèvement 2 pourrait ne comprendre que les premier 25 et deuxième 40 organes de transfert décrits précédemment ou encore un seul d'entre eux. Au moins un organe de transfert est prévu, de préférence, dans le compartiment inférieur, en aval du filtre par rapport à l'ouverture d'accès, afin d'éviter une occlusion du port de connexion par un corps solide contenu dans la matière biologique ou un prélèvement d'un tel corps solide avec les micro-organismes.

En relation avec les figures 2 à 4, un procédé de prélèvement de microbiote contenu dans une matière biologique mettant en œuvre le système de prélèvement est maintenant décrit.

Sur la figure 2, de la matière fécale est collectée en plaçant le conteneur 5 du dispositif de prélèvement 2 sur le siège des toilettes 3 avec les ailes reposant sur le rebord du siège de toilettes 3. En variante, la matière fécale pourrait être déposée de toute autre manière appropriée dans l'espace intérieur 7 du conteneur 5. Au cours de cette étape de collecte, le tube 27 peut être séparé du conteneur 5.

Sur la figure 3, une fois de la matière fécale reçue sur le filtre 20 dans l'espace intérieur 7 du corps 6, l'espace intérieur 7 est fermé en vissant le couvercle 30 sur le col 10 du conteneur 5. L'organe de recueil 25, la valve anti-retour 40 et les deux organes de prélèvement 45, 48 sont dans l'état fermé. Un opérateur peut alors procéder à la mise en anaérobiose de l'espace intérieur 7 du corps 6 du conteneur 5 en comprimant le corps 6 du conteneur 5 faisant ainsi passer automatiquement la valve anti-retour 40 dans l'état ouvert. En particulier, la paroi transversale 31 du couvercle 30 au niveau des évidements 34 forme une partie d'appui contre laquelle le corps 6 du conteneur 5 peut être pressé lorsque le couvercle 30 est monté sur le col 10 du conteneur 5. Ce faisant, une partie des gaz contenus dans l'espace intérieur 7 du corps 6 du conteneur 5, et en particulier l'oxygène, est évacuée au travers de la valve anti-retour 40 ménagée dans la partie d'appui du couvercle 30. La compression du corps 6 peut être améliorée par une préhension ou un appui sur un organe de préhension, tel qu'une plaque rigide ou une poignée, agencé sur un fond 9 à l'opposé du bord supérieur 8. Une fois que la compression du corps 6 du conteneur 5 est arrêtée, la valve anti-retour 40 repasse automatiquement dans l'état fermé pour maintenir les conditions anaérobies dans l'espace intérieur 7 du corps 6.

Selon l'application considérée, la mise en anaérobiose peut être complétée par une injection de gaz inerte dans l'espace intérieur 7 du corps 6 du conteneur 5 par l'intermédiaire de l'un des organes de transfert 25, 40, 45, 48.

Après un possible temps d'attente dans des conditions déterminées et un éventuel contrôle visuel, la matière fécale est mise en suspension en reliant un réservoir de fluide diluant, tel qu'une poche d'alimentation en fluide diluant ou un flacon de fluide diluant, au port de connexion supérieur supplémentaire 46 de l'organe d'alimentation 45 sur le couvercle 30 du dispositif de prélèvement 2. Le port de connexion supérieur supplémentaire 46 passe à l'état ouvert de telle manière que le fluide diluant est introduit dans l'espace intérieur 7 du corps 6 du conteneur 5. La matière biologique et le fluide diluant sont ensuite mélangés par pression sur le corps 6 du conteneur 5. L'homogénéité de la suspension est améliorée par son passage au travers du filtre 20.

Préalablement à la mise en suspension de la matière fécale, des analyses complémentaires peuvent être pratiquées sur un échantillon prélevé par l'intermédiaire d'un tube d'analyse relié au port de connexion supérieur supplémentaire 49 de l'organe de prélèvement 48 sur le couvercle 30 du dispositif de prélèvement 2. En variante, le prélèvement d'un tel échantillon peut être réalisé simultanément ou postérieurement à la mise en suspension.

Pour conditionner le microbiote sous forme d'inoculum prêt à l'emploi, le tube 27 ou une tubulure de distribution peut être reliée au port de connexion inférieur 26 de l'organe de recueil 25 pour prélever le microbiote contenu dans la matière fécale dans une ou plusieurs poches de recueil connectées respectivement à une ou plusieurs conduites de la tubulure de distribution.

Le microbiote peut ensuite subir toute opération appropriée pour sa conservation, son transport éventuel et sa transplantation.

## Revendications

1. Procédé de prélèvement de micro-organismes contenus dans une matière biologique, le procédé de prélèvement mettant en œuvre un dispositif de prélèvement (2) comprenant :
- un conteneur (5) comprenant un corps (6) qui comporte un espace intérieur (7) adapté pour recevoir la matière biologique, et un col (10) qui délimite une ouverture d'accès (12) à l'espace intérieur (7) du corps (6), le corps (6) du conteneur (5) étant constitué d'une poche souple,
- un couvercle (30) adapté pour être monté de manière amovible et étanche sur le col (10) du conteneur (5) de manière à obturer l'ouverture d'accès (12) du col (10) et à fermer l'espace intérieur (7) du corps (6),
dans lequel au moins l'un parmi le conteneur (5) et le couvercle (30) est pourvu d'au moins un organe de transfert (25, 40, 45, 48) comportant intérieurement un passage entre l'espace intérieur (7) et un environnement extérieur, l'organe de transfert (25, 40, 45, 48) présentant un état ouvert dans lequel ledit organe de transfert (25, 40, 45, 48) établit une communication avec l'espace intérieur (7) au travers du passage, et un état fermé dans lequel ledit organe de transfert (25, 40, 45, 48) empêche toute communication avec l'espace intérieur (7) au travers du passage, ledit au moins un organe de transfert (25, 40, 45, 48) comprenant un organe d'évacuation (40) adapté pour, dans l'état ouvert, évacuer au moins une partie des gaz contenus dans l'espace intérieur (7) du corps (6) du conteneur (5),
le procédé de prélèvement comprenant les étapes consistant à :
- collecter la matière biologique dans l'espace intérieur (7) du corps (6) du conteneur (5), et fermer l'espace intérieur (7) en montant le couvercle (30) sur le col (10) du conteneur (5),
- mettre l'espace intérieur (7) du corps (6) du conteneur (5) en anaérobiose en mettant l'organe d'évacuation (40) dans l'état ouvert, en comprimant le corps (6) du conteneur (5) de manière à évacuer au moins une partie des gaz contenus dans l'espace intérieur (7) du corps (6) du conteneur (5) et en mettant l'organe d'évacuation (40) dans l'état fermé.

2. Procédé de prélèvement selon la revendication 1, dans lequel l'organe d'évacuation (40) présente des faces intérieure et extérieure opposées, l'organe d'évacuation étant dans l'état fermé au repos et passant à l'état ouvert lorsqu'une différence de pression est appliquée entre les faces intérieure et extérieure,
le procédé de prélèvement prévoyant, au cours de l'étape consistant à mettre l'espace intérieur (7) du corps (6) du conteneur (5) en anaérobiose, de mettre automatiquement l'organe d'évacuation (40) dans l'état ouvert en comprimant le corps (6) du conteneur (5) puis de mettre automatiquement l'organe d'évacuation (40) dans l'état fermé en arrêtant de comprimer le corps (6) du conteneur (5).

3. Procédé de prélèvement (2) selon l'une quelconque des revendications 1 à 2, dans lequel le couvercle (30) comporte une partie d'appui (31) présentant une rigidité d'ensemble et dans lequel l'organe d'évacuation (40) est ménagé dans la partie d'appui (31) du couvercle (30),
le procédé de prélèvement prévoyant, au cours de l'étape consistant à mettre l'espace intérieur (7) du corps (6) du conteneur (5) en anaérobiose, de presser le corps (6) du conteneur (5) contre la partie d'appui (31) du couvercle (30).

4. Procédé de prélèvement selon l'une quelconque des revendications 1 à 3, comprenant en outre l'étape consistant à mettre en suspension la matière biologique en introduisant un fluide diluant dans l'espace intérieur (7) du corps (6) du conteneur (5) par l'intermédiaire dudit au moins un organe de transfert (25, 40, 45, 48) dans l'état ouvert, et en mélangeant la matière biologique et le fluide diluant par pression sur le corps (6) du conteneur (5).

5. Procédé de prélèvement selon la revendication 4, comprenant en outre l'étape consistant à prélever les micro-organismes en recueillant au moins une partie de la matière biologique mise en suspension par l'intermédiaire dudit au moins un organe de transfert (25, 40, 45, 48) dans l'état ouvert.

6. Procédé de prélèvement selon l'une quelconque des revendications 1 à 5, prévoyant, au cours de l'étape consistant à collecter la matière biologique dans l'espace intérieur (7) du corps (6) du conteneur (5), de collecter directement de la matière fécale en plaçant le dispositif de prélèvement sur un siège de toilettes (3).

7. Dispositif de prélèvement (2) de micro-organismes contenus dans une matière biologique, le dispositif de prélèvement (2) comprenant :
- un conteneur (5) comprenant un corps (6) qui comporte un espace intérieur (7) adapté pour recevoir la matière biologique, et un col (10) qui délimite une ouverture d'accès (12) à l'espace intérieur (7) du corps (6), le corps (6) du conteneur (5) étant constitué d'une poche souple,
- un couvercle (30) adapté pour être monté de manière amovible et étanche sur le col (10) du conteneur (5) de manière à obturer l'ouverture d'accès (12) du col (10) et à fermer l'espace intérieur (7) du corps (6),
dans lequel au moins l'un parmi le conteneur (5) et le couvercle (30) est pourvu d'au moins un organe de transfert (25, 40, 45, 48) comportant intérieurement un passage entre l'espace intérieur (7) et un environnement extérieur, l'organe de transfert (25, 40, 45, 48) présentant un état ouvert dans lequel ledit organe de transfert (25, 40, 45, 48) établit une communication avec l'espace intérieur (7) au travers du passage, et un état fermé dans lequel ledit organe de transfert (25, 40, 45, 48) empêche toute communication avec l'espace intérieur (7) au travers du passage,
le dispositif de prélèvement (2) étant **caractérisé en ce que** ledit au moins un organe de transfert (25, 40, 45, 48) comprend un organe d'évacuation (40) présentant des faces intérieure et extérieure opposées, l'organe d'évacuation étant dans l'état fermé au repos et passant à l'état ouvert lorsqu'une différence de pression est appliquée entre les faces intérieure et extérieure, de manière à mettre automatiquement l'organe d'évacuation (40) dans l'état ouvert en comprimant le corps (6) du conteneur (5) pour évacuer au moins une partie des gaz contenus dans l'espace intérieur (7) du corps (6) du conteneur (5) et mettre l'espace intérieur (7) du corps (6) du conteneur (5) en anaérobiose, et à mettre automatiquement l'organe d'évacuation (40) dans l'état fermé en arrêtant de comprimer le corps (6) du conteneur (5).

8. Dispositif de prélèvement (2) selon la revendication 7, dans lequel le couvercle (30) comporte une partie d'appui (31) présentant une rigidité d'ensemble et dans lequel l'organe d'évacuation (40) est ménagé dans la partie d'appui (31) du couvercle (30), de manière à pouvoir presser le corps (6) du conteneur (5) contre la partie d'appui (31) du couvercle (30) pour mettre l'espace intérieur (7) du corps (6) du conteneur (5) en anaérobiose.

9. Dispositif de prélèvement (2) selon l'une quelconques des revendications 7 et 8, dans lequel l'organe d'évacuation (40) comprend une membrane de retenue microporeuse disposée dans le passage et adaptée pour retenir la matière biologique tout en laissant passer les gaz.

10. Dispositif de prélèvement (2) selon l'une quelconque des revendications 7 à 9, dans lequel ledit au moins un organe de transfert (25, 40, 45, 48) comprend un port de connexion (26, 41, 46, 49) ménagé au travers de l'un parmi le conteneur (5) et le couvercle (30) et comportant intérieurement au moins une partie du passage, et un élément d'obturation (47) déplaçable par rapport au port de connexion (26, 41, 46, 49) entre une position d'obturation, dans laquelle ledit élément d'obturation (47) empêche toute communication au travers du passage, et une position de libération dans laquelle ledit élément d'obturation (47) autorise la communication au travers du passage.

11. Dispositif de prélèvement (2) selon la revendication 10, dans lequel l'élément d'obturation (47) est monté dans le passage du port de connexion (26, 46), l'élément d'obturation (47) étant sollicité vers la position d'obturation et étant déplaçable vers la position de libération.

12. Dispositif de prélèvement (2) selon l'une quelconque des revendications 10 et 11, dans lequel ledit au moins un organe de transfert (25, 40) comprend au moins un tube (27, 42) s'étendant depuis le port de connexion (26, 41) et comportant intérieurement une partie du passage.

13. Dispositif de prélèvement (2) selon la revendication 12, dans lequel ledit au moins un organe de transfert (25) comprend au moins une vanne (28) montée sur le tube (27), la vanne (28) étant déplaçable entre une position fermée, dans laquelle ladite vanne (28) empêche toute communication au travers du passage, et une position ouverte dans laquelle ladite (28) vanne autorise la communication au travers du passage.

14. Dispositif de prélèvement (2) selon l'une quelconque des revendications 12 et 13 lorsqu'elle dépend de la revendication 11, dans lequel le tube (27) comporte un port de connexion complémentaire pourvu d'un élément de libération et adapté pour être connecté de manière amovible au port de connexion (26) de telle manière que l'élément de libération déplace l'élément d'obturation vers la position de libération.

15. Dispositif de prélèvement (2) selon l'une quelconque des revendications 7 à 14, comprenant en outre un filtre (20) solidarisé au conteneur (5) de manière à définir, dans l'espace intérieur (7), un compartiment supérieur dans lequel débouche l'ouverture d'accès (12), et un compartiment inférieur, le filtre (20) présentant des pores compris entre 0,1 mm et 1,5 mm.

16. Kit de prélèvement (1) comprenant :
- un dispositif de prélèvement (2) selon l'une quelconque des revendications 7 à 15, et
- au moins un dispositif annexe choisi parmi un dispositif d'alimentation adapté pour alimenter l'espace intérieur (7) en fluide, et un dispositif de réception adapté pour recevoir un fluide contenu dans l'espace intérieur (7), le dispositif annexe étant notamment choisi parmi un réservoir de fluide diluant, un tube d'analyse, une tubulure de distribution et une poche de recueil de micro-organismes.

17. Kit de prélèvement (1) selon la revendication 16 lorsqu'elle dépend de la revendication 11, dans lequel le dispositif annexe comporte un port de connexion complémentaire pourvu d'un élément de libération et adapté pour être connecté de manière amovible au port de connexion (26, 46) de l'organe de transfert (25, 45) du dispositif de prélèvement (2) de telle manière que l'élément de libération déplace l'élément d'obturation vers la position de libération.

## Patentansprüche

1. Verfahren zur Entnahme von Mikroorganismen, die in einem biologischen Material enthalten sind, wobei bei dem Entnahmeverfahren eine Entnahmevorrichtung (2) Anwendung findet, enthaltend:
- einen Behälter (5) mit einem Körper (6), der einen Innenraum (7) aufweist, der zur Aufnahme des biologischen Materials geeignet ist, und mit einem Hals (10), der eine Zugangsöffnung (12) zum Innenraum (7) des Körpers (6) begrenzt, wobei der Körper (6) des Behälters (5) aus einem nachgiebigen Beutel besteht,
- einen Deckel (30), der dazu geeignet ist, abnehmbar und abdichtend am Hals (10) des Behälters (5) angebracht zu werden, um die Zugangsöffnung (12) des Halses (10) zu schließen und den Innenraum (7) des Körpers (6) zu verschließen,
wobei zumindest eines aus Behälter (5) und Deckel (30) mit zumindest einem Transferelement (25, 40, 45, 48) versehen ist, das im Inneren einen Durchgang zwischen dem Innenraum (7) und einer äußeren Umgebung aufweist, wobei das Transferelement (25, 40, 45, 48) einen offenen Zustand aufweist, in dem das Transferelement (25, 40, 45, 48) eine Kommunikation mit dem Innenraum (7) durch den Durchgang herstellt, sowie einen geschlossenen Zustand, in dem das Transferelement (25, 40, 45, 48) jegliche Kommunikation mit dem Innenraum (7) durch den Durchgang verhindert, wobei das zumindest eine Transferelement (25, 40, 45, 48) ein Ableitelement (40) enthält, das dazu geeignet ist, in dem offenen Zustand zumindest einen Teil der in dem Innenraum (7) des Körpers (6) des Behälters (5) enthaltenen Gase abzuleiten,
wobei das Entnahmeverfahren die folgenden Schritte umfasst:
- Sammeln des biologischen Materials im Innenraum (7) des Körpers (6) des Behälters (5), und Verschließen des Innenraums (7) durch Anbringen des Deckels (30) am Hals (10) des Behälters (5),
- Versetzen des Innenraums (7) des Körpers (6) des Behälters (5) in Anaerobiose durch Versetzen des Ableitelements (40) in den offenen Zustand, durch Zusammendrücken des Körpers (6) des Behälters (5), um zumindest einen Teil der im Innenraum (7) des Körpers (6) des Behälters (5) enthaltenen Gase abzuleiten, und durch Versetzen des Ableitelements (40) in den geschlossenen Zustand.

2. Entnahmeverfahren nach Anspruch 1,
wobei das Ableitelement (40) gegenüberliegend eine Innen- und eine Außenfläche aufweist, wobei sich das Ableitelement im Ruhezustand im geschlossenen Zustand befindet und dann in den offenen Zustand übergeht, wenn eine Druckdifferenz zwischen der Innen- und der Außenfläche aufgebracht wird,
wobei das Entnahmeverfahren vorsieht, in dem Schritt des Versetzens von dem Innenraum (7) des Körpers (6) des Behälters (5) in Anaerobiose, das Ableitelement (40) automatisch in den offenen Zustand zu versetzen, indem der Körper (6) des Behälters (5) zusammengedrückt wird, und dann das Ableitelement (40) automatisch in den geschlossenen Zustand zu versetzen, indem das Zusammendrücken des Körpers (6) des Behälters (5) unterbrochen wird.

3. Entnahmeverfahren nach einem der Ansprüche 1 bis 2,
wobei der Deckel (30) einen Stützabschnitt (31) mit einer Gesamtsteifigkeit aufweist und wobei das Ableitelement (40) im Stützabschnitt (31) des Deckels (30) ausgebildet ist,
wobei das Entnahmeverfahren vorsieht, in dem Schritt des Versetzens von dem Innenraum (7) des Körpers (6) des Behälters (5) in Anaerobiose, den Körper (6) des Behälters (5) an den Stützabschnitt (31) des Deckels (30) anzudrücken.

4. Entnahmeverfahren nach einem der Ansprüche 1 bis 3,
ferner umfassend den Schritt des Suspendierens des biologischen Materials durch Einleiten eines Verdünnungsfluids in den Innenraum (7) des Körpers (6) des Behälters (5) mittels des zumindest einen Transferelements (25, 40, 45, 48) im offenen Zustand und durch Mischen des biologischen Materials und des Verdünnungsfluids durch Drücken auf den Körper (6) des Behälters (5).

5. Entnahmeverfahren nach Anspruch 4,
ferner umfassend den Schritt der Entnahme der Mikroorganismen durch Aufnehmen zumindest eines Teils des suspendierten biologischen Materials mittels des zumindest einen Transferelements (25, 40, 45, 48) im offenen Zustand.

6. Entnahmeverfahren nach einem der Ansprüche 1 bis 5,
wobei während des Schrittes des Sammelns des biologischen Materials im Innenraum (7) des Körpers (6) des Behälters (5) vorgesehen ist, fäkales Material direkt zu sammeln, indem die Entnahmevorrichtung auf einen Toilettensitz (3) gestellt wird.

7. Entnahmevorrichtung (2) für Mikroorganismen, die in einem biologischen Material enthalten sind, wobei die Entnahmevorrichtung (2) enthält:
- einen Behälter (5) mit einem Körper (6), der einen Innenraum (7) aufweist, der zur Aufnahme des biologischen Materials geeignet ist, und mit einem Hals (10), der eine Zugangsöffnung (12) zum Innenraum (7) des Körpers (6) begrenzt, wobei der Körper (6) des Behälters (5) aus einem nachgiebigen Beutel besteht,
- einen Deckel (30), der geeignet ist, abnehmbar und abdichtend am Hals (10) des Behälters (5) angebracht zu werden, um die Zugangsöffnung (12) des Halses (10) zu schließen und den Innenraum (7) des Körpers (6) zu verschließen,
wobei zumindest eines aus Behälter (5) und Deckel (30) mit zumindest einem Transferelement (25, 40, 45, 48) versehen ist, das im Inneren einen Durchgang zwischen dem Innenraum (7) und einer äußeren Umgebung aufweist, wobei das Transferelement (25, 40, 45, 48) einen offenen Zustand aufweist, in dem das Transferelement (25, 40, 45, 48) eine Kommunikation mit dem Innenraum (7) durch den Durchgang herstellt, sowie einen geschlossenen Zustand, in dem das Transferelement (25, 40, 45, 48) jegliche Kommunikation mit dem Innenraum (7) durch den Durchgang verhindert, wobei die Entnahmevorrichtung (2) **dadurch gekennzeichnet ist, dass** das zumindest eine Transferelement (25, 40, 45, 48) ein Ableitelement (40) enthält, das gegenüberliegend eine Innen- und eine Außenfläche aufweist, wobei sich das Ableitelement im Ruhezustand im geschlossenen Zustand befindet und dann in den offenen Zustand übergeht, wenn eine Druckdifferenz zwischen der Innen- und der Außenfläche aufgebracht wird, so dass das Ableitelement (40) automatisch in den offenen Zustand versetzt wird, indem der Körper (6) des Behälters (5) zusammengedrückt wird, um zumindest einen Teil der im Innenraum (7) des Körpers (6) des Behälters (5) enthaltenen Gase abzuleiten und den Innenraum (7) des Körpers (6) des Behälters (5) in Anaerobiose zu versetzen, und dass das Ableitelement (40) automatisch in den geschlossenen Zustand versetzt wird, indem das Zusammendrücken des Körpers (6) des Behälters (5) unterbrochen wird.

8. Entnahmevorrichtung (2) nach Anspruch 7,
wobei der Deckel (30) einen Stützabschnitt (31) mit einer Gesamtsteifigkeit aufweist und wobei das Ableitelement (40) im Stützabschnitt (31) des Deckels (30) ausgebildet ist, so dass der Körper (6) des Behälters (5) an den Stützabschnitt (31) des Deckels (30) angedrückt werden kann, um den Innenraum (7) des Körpers (6) des Behälters (5) in Anaerobiose zu versetzen.

9. Entnahmevorrichtung (2) nach einem der Ansprüche 7 und 8,
wobei das Ableitelement (40) eine mikroporöse Rückhaltemembran enthält, die in dem Durchgang angeordnet und dazu geeignet ist, biologisches Material zurückzuhalten, während sie Gase passieren lässt.

10. Entnahmevorrichtung (2) nach einem der Ansprüche 7 bis 9,
wobei das zumindest eine Transferelement (25, 40, 45, 48) eine Anschlussöffnung (26, 41, 46, 49) aufweist, die entweder durch den Behälter (5) oder den Deckel (30) hindurch vorgesehen ist und im Inneren zumindest einen Teil des Durchgangs aufweist, sowie ein Verschlusselement (47), das zwischen einer Verschlussposition, in der das Verschlusselement (47) jegliche Kommunikation durch den Durchgang verhindert, und einer Freigabeposition, in der das Verschlusselement (47) eine Kommunikation durch den Durchgang ermöglicht, relativ zu der Anschlussöffnung (26, 41, 46, 49) beweglich ist.

11. Entnahmevorrichtung (2) nach Anspruch 10,
wobei das Verschlusselement (47) in dem Durchgang der Anschlussöffnung (26, 46) angebracht ist, wobei das Verschlusselement (47) in die Verschlussposition beaufschlagt wird und in die Freigabeposition bewegt werden kann.

12. Entnahmevorrichtung (2) nach einem der Ansprüche 10 und 11,
wobei das zumindest eine Transferelement (25, 40) zumindest eine Röhre (27, 42) aufweist, die sich von der Anschlussöffnung (26, 41) ausgehend erstreckt und im Inneren einen Teil des Durchgangs enthält.

13. Entnahmevorrichtung (2) nach Anspruch 12,
wobei das zumindest eine Transferelement (25) zumindest ein Ventil (28) aufweist, das an der Röhre (27) angebracht ist, wobei das Ventil (28) zwischen einer geschlossenen Position, in der das Ventil (28) jegliche Kommunikation durch den Durchgang verhindert, und einer offenen Position, in der das Ventil (28) die Kommunikation durch den Durchgang zulässt, beweglich ist.

14. Entnahmevorrichtung (2) nach einem der Ansprüche 12 und 13 in Abhängigkeit von Anspruch 11,
wobei die Röhre (27) eine komplementäre Anschlussöffnung aufweist, die mit einem Freigabeelement versehen ist und so mit der Anschlussöffnung (26) lösbar verbunden werden kann, dass das Freigabeelement das Verschlusselement in die Freigabeposition bewegt.

15. Entnahmevorrichtung (2) nach einem der Ansprüche 7 bis 14,
ferner enthaltend einen Filter (20), der fest mit dem Behälter (5) verbunden ist, so dass in dem Innenraum (7) ein oberes Fach, in das die Zugangsöffnung (12) mündet, und ein unteres Fach definiert werden, wobei der Filter (20) Poren zwischen 0,1 mm und 1,5 mm aufweist.

16. Entnahme-Set (1), enthaltend:
- eine Entnahmevorrichtung (2) nach einem der Ansprüche 7 bis 15,
- zumindest eine Zusatzvorrichtung, die ausgewählt ist aus einer Versorgungsvorrichtung zum Versorgen des Innenraums (7) mit Fluid und einer Aufnahmevorrichtung zum Aufnehmen von im Innenraum (7) enthaltenem Fluid, wobei die Zusatzvorrichtung insbesondere ausgewählt ist aus einem Vorratsbehälter für Verdünnungsfluid, einer Analyseröhre, einem Ausgabestutzen und einem Sammelbeutel für Mikroorganismen.

17. Entnahme-Set (1) nach Anspruch 16 in Abhängigkeit von Anspruch 11, wobei die Zusatzvorrichtung eine komplementäre Anschlussöffnung aufweist, die mit einem Freigabeelement versehen ist und mit der Anschlussöffnung (26, 46) des Transferelements (25, 45) der Entnahmevorrichtung (2) derart lösbar verbindbar ist, dass das Freigabeelement das Verschlusselement in die Freigabeposition bewegt.

## Claims

1. A sampling method for sampling microorganisms contained in biological matter, the sampling method employing a sampling device (2) comprising:
- a container (5) comprising a body (6) which comprises an internal space (7) configured to receive the biological matter, and a neck (10) which delimits an access opening (12) to the internal space (7) of the body (6), the body (6) of the container (5) being constituted by a flexible bag,
- a cover (30) configured to be removably and sealingly mounted on the neck (10) of the container (5) so as to obturate the access opening (12) of the neck (10) and close the internal space (7) of the body (6),
wherein at least one of the container (5) and the cover (30) is provided with at least one transfer member (25, 40, 45, 48) internally comprising a passage between the internal space (7) and an external environment, the transfer member (25, 40, 45, 48) having an open state in which said transfer member (25, 40, 45, 48) establishes communication with the internal space (7) through the passage, and a closed state in which said transfer member (25, 40, 45, 48) prevents all communication with the internal space (7) through the passage, said at least one transfer member (25, 40, 45, 48) comprising an evacuation member (40) configured for evacuating, in the open state, at least part of the gases contained in the internal space (7) of the body (6) of the container (5),
the sampling method comprising the steps consisting of:
- collecting the biological matter in the internal space (7) of the body (6) of the container (5), and closing the internal space (7) by mounting the cover (30) on the neck (10) of the container (5),
- putting the internal space (7) of the body (6) of the container (5) under anaerobiosis by putting the evacuation member (40) in the open state, by compressing the body (6) of the container (5) so as to evacuate at least part of the gases contained in the internal space (7) of the body (6) of the container (5) by putting the evacuation member (40) in the closed state.

2. A sampling method according to claim 1, wherein the evacuation member (40) has opposite internal and external faces, the evacuation member being in the closed state when at rest and passing into the open state when a difference of pressure is applied between the internal and external faces,
the sampling method providing, during the step consisting of putting the internal space (7) of the body (6) of the container (5) under anaerobiosis, of automatically putting the evacuation member (40) in the open state by compressing the body (6) of the container (5) then automatically putting the evacuation member (40) in the closed state by stopping compressing the body (6) of the container (5).

3. A sampling method (2) according to any one of claims 1 to 2, wherein the cover (30) comprises a bearing part (31) having overall rigidity and wherein the evacuation member (40) is provided in the bearing part (31) of the cover (30),
the sampling method providing, during the step consisting of putting the internal space (7) of the body (6) of the container (5) under anaerobiosis, for pressing the body (6) of the container (5) against the bearing part (31) of the cover (30).

4. A sampling method according to any one of claims 1 to 3, further comprising the step consisting of suspending the biological matter by introducing a diluent fluid into the internal space (7) of the body (6) of the container (5) via said at least one transfer member (25, 40, 45, 48) in the open state, and by mixing the biological matter and the diluent fluid by pressing on the body (6) of the container (5).

5. A sampling method according to claim 4, further comprising the step consisting of sampling the microorganisms by collecting at least some of the biological matter suspended via said at least one transfer member (25, 40, 45, 48) in the open state.

6. A sampling method according to any one of claims 1 to 5, providing, during the step consisting of collecting the biological matter in the internal space (7) of the body (6) of the container (5), for directly collecting the faecal matter by placing the sampling device on a toilet seat (3).

7. A sampling device (2) for sampling microorganisms contained in biological matter, the sampling device (2) comprising:
- a container (5) comprising a body (6) which comprises an internal space (7) configured to receive the biological matter, and a neck (10) which delimits an access opening (12) to the internal space (7) of the body (6), the body (6) of the container (5) being constituted by a flexible bag,
- a cover (30) configured to be removably and sealingly mounted on the neck (10) of the container (5) so as to obturate the access opening (12) of the neck (10) and close the internal space (7) of the body (6),
wherein at least one of the container (5) and the cover (30) is provided with at least one transfer member (25, 40, 45, 48) internally comprising a passage between the internal space (7) and an external environment, the transfer member (25, 40, 45, 48) having an open state in which said transfer member (25, 40, 45, 48) establishes communication with the internal space (7) through the passage, and a closed state in which said transfer member (25, 40, 45, 48) prevents all communication with the internal space (7) through the passage,
the sampling device (2) being **characterized in that** said at least one transfer member (25, 40, 45, 48) comprises an evacuation member (40) having opposite internal and external faces, the evacuation member being in the closed state when at rest and passing into the open state when a difference of pressure is applied between the internal and external faces, so as to automatically put the evacuation member (40) in the open state by compressing the body (6) of the container (5) to evacuate at least part of the gases contained in the internal space (7) of the body (6) of the container (5) and place the internal space (7) of the body (6) of the container (5) under anaerobiosis, and to automatically put the evacuation member (40) in the closed state by stopping compressing the body (6) of the container (5).

8. A sampling device (2) according to claim 7, wherein the cover (30) comprises a bearing part (31) having overall rigidity and wherein the evacuation member (40) is provided in the bearing part (31) of the cover (30), so as to be able to press the body (6) of the container (5) against the bearing part (31) of the cover (30) to put the internal space (7) of the body (6) of the container (5) under anaerobiosis.

9. A sampling device (2) according to any one of claims 7 and 8, wherein the evacuation member (40) comprises a microporous retaining membrane disposed in the passage and configured to retain the biological matter while allowing gases to pass.

10. A sampling device (2) according to any one of claims 7 to 9, wherein said at least one transfer member (25, 40, 45, 48) comprises a connection port (26, 41, 46, 49) provided through one of the container (5) and the cover (30) and internally comprising at least part of the passage, and an obturating member (47) movable relative to the connection port (26, 41, 46, 49) between an obturating position, in which said obturating member (47) prevents all communication through the passage, and a freeing position in which said obturating member (47) allows communication through the passage.

11. A sampling device (2) according to claim 10, wherein the obturating member (47) is mounted in the passage of the connection port (26, 46), the obturating member (47) being urged towards its obturating position and being movable towards the freeing position.

12. A sampling device (2) according to any one of claims 10 and 11, wherein said at least one transfer member (25, 40) comprises at least one tube (27, 42) extending from the connection port (26, 41) and internally comprising part of the passage.

13. A sampling device (2) according to claim 12, wherein said at least one transfer member (25) comprises at least one valve (28) mounted on the tube (27), the valve (28) being movable between a closed position, in which said valve (28) prevents all communication through the passage, and an open position in which said valve (28) allows the communication through the passage.

14. A sampling device (2) according to any one of claims 12 to 13, when it is dependent from claim 11, wherein the tube (27) comprises a complementary connection port provided with a freeing member and which is configured to be removably connected to the connection port (26) such that the freeing member moves the obturating member towards the freeing position.

15. A sampling device (2) according to any one of claims 7 to 14, further comprising a filter (20) attached to the container (5) so as to define, in the internal space (7), an upper compartment into which the access opening (12) opens, and a lower compartment, the filter (20) having pores comprised between 0.1 mm and 1.5 mm.

16. A sampling kit (1) comprising:
- a sampling device (2) according to any one of claims 7 to 15, and
- at least one ancillary device chosen from a supply device configured to supply the internal space (7) with fluid, and a receiving device configured to receive a fluid contained in the internal space (7), the ancillary device being in particular chosen from a reservoir of diluent fluid, an analysis tube, a distribution pipe and a collecting bag for microorganisms.

17. A sampling kit (1) according to claim 16 when it is dependent from claim 11, wherein the ancillary device comprises a complementary connection port provided with a freeing member and which is configured to be removably connected to the connection port (26, 46) of the transfer member (25, 45) of the sampling device (2) such that the freeing member moves the obturating member towards the freeing position.
